# EUROPEAN PATENT SPECIFICATION

(11) **EP 1 005 843 B1**
(45) Date of publication and mention of the grant of the patent: **26.01.2005**
(21) Application number: 99120028.8
(22) Date of filing: 18.10.1999
(51) Int. Cl.: A61F 2/06

(54) **Controlled detachment stents**
Mit einer kontrollierten Trennmöglichkeit verbundene Stents
Ensemble de stents à détachement controlé

(30) Priority: 03.12.1998 US 204830
(43) Date of publication of application: 07.06.2000
(73) Proprietor: Medinol Ltd., Tel Aviv 61581 (IL)
(72) Inventor: Richter, Jacob, Ramat Hasharon 47226 (IL)
(74) Representative: Altenburg, Udo, Dipl.-Phys.

(56) References cited:
- WO-A-97/37617
- US-A- 5 591 223
- US-A- 5 723 003

## Description

### FIELD OF THE INVENTION

The invention relates generally to stents, which are endoprostheses implanted into vessels within the body, such as a blood vessels, to support and hold open the vessels, or to secure and support other endoprostheses in vessels.

### BACKGROUND OF THE INVENTION

Various stents are known in the art. Typically stents are generally tubular in shape, and are expandable from a relatively small, unexpanded diameter to a larger, expanded diameter. For implantation, the stent is typically mounted on the end of a catheter, with the stent being held on the catheter at its relatively small, unexpanded diameter. By the catheter, the unexpanded stent is directed through the lumen to the intended implantation site. Once the stent is at the intended implantation site, it is expanded, typically either by an internal force, for example by inflating a balloon on the inside of the stent, or by allowing the stent to self-expand, for example by removing a sleeve from around a self-expanding stent, allowing the stent to expand outwardly. In either case, the expanded stent resists the tendency of the vessel to narrow, thereby maintaining the vessel's patency.

Some examples of patents relating to stents include U.S. Patent No. 4,733,665 to Palmaz; U.S. Patent No. 4,800,882 and 5,282,824 to Gianturco; U.S. Patent Nos. 4,856,516 and 5,116,365 to Hillstead; U.S. Patent Nos. 4,886,062 and 4,969,458 to Wiktor; U.S. Patent No. 5,019,090 to Pinchuk; U.S. Patent No. 5,102,417 to Palmaz and Schatz; U.S. Patent No. 5,104,404 to Wolff; U.S. Patent No. 5,161,547 to Tower; U.S. Patent No. 5,383,892 to Cardon et al.; U.S. Patent No. 5,449,373 to Pinchasik et al.; and U.S. Patent No. 5,733,303 to Israel et al.

WO 97/37617 discloses a stent consisting of a tubular member interconnected to another tubular member with a flexible member, each stent made from a flat sheet assembled together through a technique such as surface fusing. Preferably, the stent is made up of a plurality of spaced rows of slots with spaces between adjacent slots within a row staggered with respect to corresponding spaces on adjacent rows.

US-A-5 591 223 describes a radially expandable endoprosthesis device that comprises an elongated sleeve member in which the radially outward expansion of the sleeve member is initially limited by connecting strips which are operatively connected to the sleeve member. The connecting strips are made of a material that ruptures in a predetermined pressure selected from a range of four to ten atmospheres, said connecting strips being selectively removable to allow further radial outward expansion.

One object of prior stent designs has been to insure that the stent has sufficient radial strength when it is expanded so that it can sufficiently support the lumen. Stents with high radial strength, however, tend also to have a higher longitudinal rigidity than the vessel in which it is implanted. When the stent has a higher longitudinal rigidity than the vessel in which it is implanted, increased trauma to the vessel may occur at the ends of the stent, due to stress concentrations on account of the mismatch in compliance between the stented and un-stented sections of the vessel.

### SUMMARY OF THE INVENTION

An object of the invention is to provide a stent that more closely matches the compliance of the vessel in which it is implanted, with relatively little or no sacrifice in radial strength, even when the stent is made very long.

This object is achieved according to the invention by a stent as defined in independent claim 1. Advantageous embodiments are depicted in the dependent claims.

In accordance with one embodiment of the invention, a stent is provided with specific "designated detachment" points, such that after the stent is deployed, and during the motion of the vessel, the stress applied on the stent will cause the stent to segment at these designated detachment points. When the designated detachment points are arranged completely around the circumference of the stent, creating a circumferential "designated detachment" zone, the detachment at the designated detachment points separates the stent into two or more separate stent segments, each able to move with the vessel independently of the other stent segments. Because each stent segment can move with the vessel independently of the other stent segments, the series of stent segments achieves greater compliance between the stented and un-stented sections of the vessel than the longer, unitary stent, and it thereby reduces stress on the vessel wall.

The stent is preferably designed such that after detachment, the ends of the stent segments created by the detachment are relatively smooth, so that they do not injure the vessel wall. Also, the stent is preferably configured such that the individual stent segments have sufficient radial strength after detachment, such that the detachment results in little or no significant reduction in the stent's resistance to compression.

The stent may be designed such that detachment occurs only after a period of cime after implantation, so that the stent will already be buried under neointima at the time of detachment. Thus, the stent segments remaining after detachment will be held in place by the neointima and will not move relative to the lumen, i.e., they will not "telescope" into one another, and they will not move away from one another, creating unsupported gaps.

A variety of mechanisms may be used to accomplish the detachment. For example, the stent may be provided at certain points or zones along its length with components having a cross-sectional area sufficiently low so that the stent segments will detach preferentially under the stress placed on the stent after implantation. Alternatively or additionally, the stent may be provided at certain points or zones along its length with components made of a material that is sufficiently weaker than elsewhere in the stent so that the stent segments will detach preferentially under the stress placed on the stent after implantation.
Alternatively or additionally, the stent may be designed such that it has a lower number of components, or struts, at the designated detachment zones, so that each such component bears more load than components elsewhere in the stent.
These components are configured to separate under the increased loads they bear when the stent is repeatedly stressed after implantation.

The factors contributing to detachment may be applied individually or in combination. For example, the designated detachment struts may have low cross-sectional areas and also may be formed of weaker material, or the designated detachment zones may have a reduced number of components, with or without the components having low cross-sectional areas and/or being formed of weaker material.

### BRIEF DESCRIPTION OF THE DRAWINGS

- Figure 1: shows a schematic diagram of a stent, generally in the form of a cylinder, having designated detachment zones between stent segments;
- Figure 2: shows a schematic diagram of the stent of Figure 1 after detachment, in which the stent has separated into a series of shorter stent segments;
- Figure 3: shows a flat layout of a stent pattern in which the components in the designated detachment zones have a cross-sectional area that is sufficiently low so that the stent segments will detach under the stress placed on the stent after implantation;
- Figure 4: shows a flat layout of the stent pattern of Figure 3, after detachment has occurred at the designated detachment zones; and
- Figure 5: shows a flat layout of a stent pattern in which the stent has a lower number of components at the designated detachment zones, so that each such component bears an increased load and separates under such increased load.

### DETAILED DESCRIPTION OF THE DRAWINGS

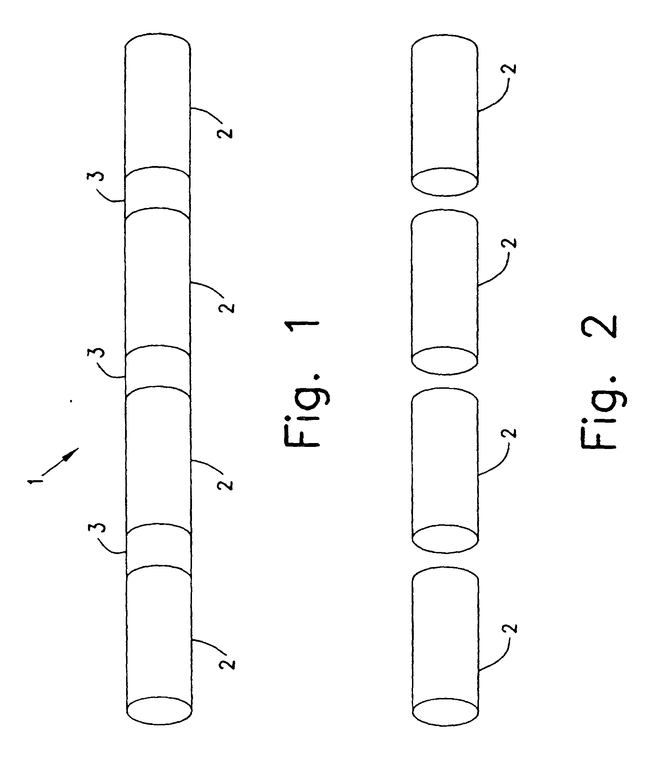

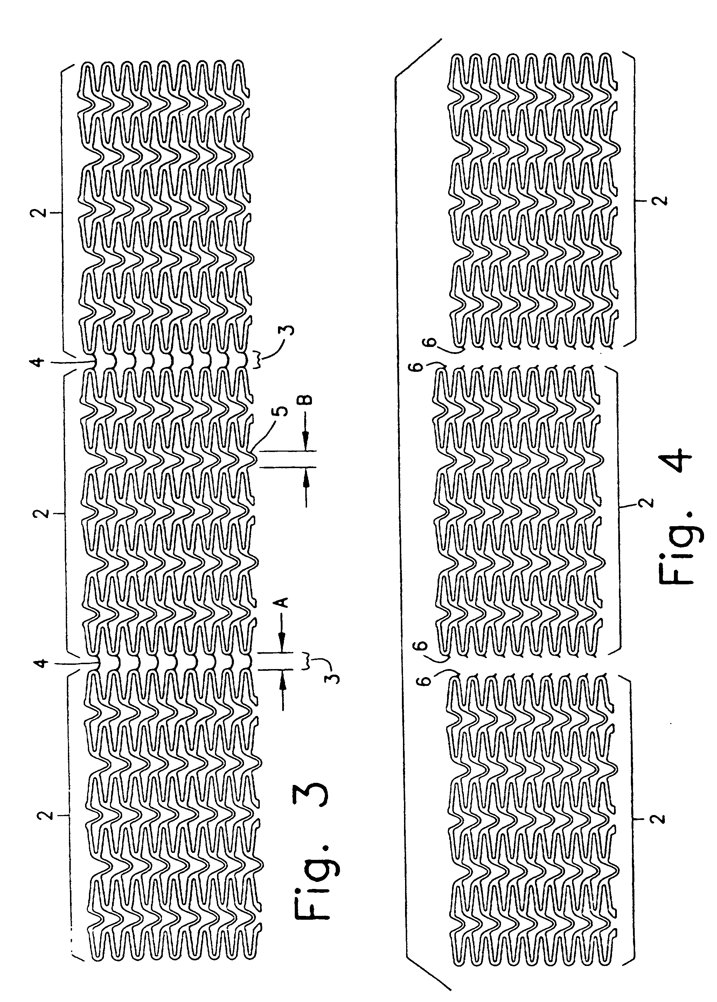

Figure 1 shows a schematic diagram of a stent 1, generally in the form of a cylinder. The stent 1 comprises a series of stent segments 2 separated by designated detachment zones 3. The designated detachment zones 3 comprise one or more designated detachment components or struts (see Figures 3 through 5).

The designated detachment zones 3 are designed such that the designated detachment components or struts separate under repeated stress placed on the stent 1 after implantation. When all of the designated detachment struts around the circumference of the stent in a particular designated detachment zone 3 separate, the stent is itself separated into a series of independent stent segments 2, as shown in Figure 2. The designated detachment zones 3 may be designed such that detachment does not occur until some time has passed after implantation, so that the stent segments 2 will already be buried under neointima at the time of detachment and therefore will not move relative to the lumen.

Persons of ordinary skill in the art will appreciate that the basic geometry of the stent segments 2 may take any suitable form, and that the stent segments 2 may be formed of any suitable material. Examples of suitable structures for the stent segments 2 include those shown in U.S. Patent No. 5,733,303 to Israel et al..

Figure 3 shows a flat layout of a stent pattern comprising stent segments 2 separated by designated detachment zones 3. In the finished stent, each stent segment 2 in this embodiment has a configuration generally corresponding to a stent configuration disclosed in U.S. Patent No. 5,733,303. The stent segments 2 are joined to each other by the designated detachment components or struts 4 in the designated detachment zones 3.

In this embodiment, each of the designated detachment struts 4 has a reduced cross-sectional area that is sufficiently low to allow separation of the designated detachment struts 4 under the stress placed on the stent after implantation. The amount of reduction of the cross-section of the detachment struts 4 as compared to, for example, the components labeled by reference numeral 5 in the stent segments 2, may be, for example, on the order of tens of percents. For example, the detachment struts 4 may be 25% to 75% thinner or narrower than the components 5.

These designated detachment struts 4 may additionally or alternatively be made of a weaker material, in order to insure appropriate separation. The weaker material may be provided either in the stock material used to form the designated detachment struts 4, or by treating the designated detachment struts 4 (or the designated detachment zones 3) after the stent has been produced, such that the treatment weakens the material of the designated detachment struts 4.

One example of a manner of providing the designated detachment struts with weaker material is to form the entire stent of NiTi and then to treat the designated detachment struts to be Martensitic while the remaining components will be in the Austenitic phase. Another example, for example in a stent made of SST, is to anneal the components in the designated detachment zones and harden the components in the stent segments.

In addition to the reduction in cross-section, the remaining geometry of the designated detachment struts may be selected to achieve the desired results. As shown in Figure 3, the width A of the row of designated detachment struts 4 may be narrower than the width of a corresponding row of components in the stent segment 2, for example the width B of the row of components labeled by reference numeral 5. This reduced width at the designated detachment zones 3 helps to insure detachment at the designated detachment zones 3 under longitudinal repeat bending. Also , the designated detachment struts 4 may be made sufficiently short to reduce the length of the free ends after separation, so as not to leave long, hanging ends after detachment. For example, the length of the designated detachment struts 4 is shorter than the length of the components 5.

Figure 4 shows a flat layout of the stent pattern of Figure 3 after detachment has occurred at the designated detachment zones 3. As shown in Figure 4, the stent after detachment comprises a series of separated and independent stent segments 2. As also seen in Figure 4, because the designated detachment struts 4 were short, the length of the free ends 6 after separation is kept to a minimum.

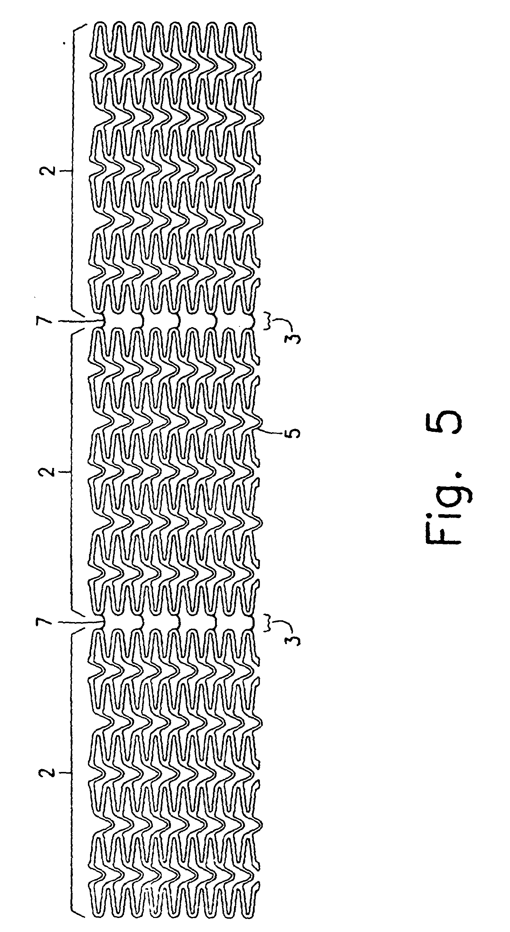

Figure 5 shows an alternative design in which, in the designated detachment zones 3, the stent is provided with a lower number of components 7 around the circumference of the stent. In the embodiment shown in Figure 5, each designated detachment zone 3 has five designated detachment struts 7 around the circumference of the stent. By comparison, the stent has nine of the components labeled as component 5 in a band of such components within the stent segments 2. Of course, different numbers of designated detachment struts and stent segment components may be used, without departing from the general concept of the invention.

The designated detachment struts 7 are configured such that they detach under the loads they bear on account of the stress placed on the stent after implantation. As shown in Figure 5, the designated detachment struts 7 may also have a reduced cross-sectional area. Also, as with the designated detachment struts in other embodiments, the designated detachment struts 7 may additionally be formed of weaker material, or the designated detachment struts 7 or zones 3 may be treated to make the material weaker after production of the stent.

The embodiments described herein are examples only, as other variations are within the scope of the invention as defined by the appended claims.

## Claims

1. A stent (1) for implantation in a vessel, wherein the stent (1) comprises a plurality of stent segments (2) and means for connecting adjacent stent segments
**characterized in that**
said connecting means are detachable, wherein said detachable connecting means comprise at least one designated detachment strut (4), wherein the designated detachment strut (4) has a cross-sectional area that is less than the cross-sectional area of a component (5) within one of said stent segments (2) and/or the designated detachment strut (4) is made of a material that is weaker than the material of a component (5) within one of said stent segments (2), so that said adjacent stent segments (2) separate from each other in response to the physiological stress placed on said detachable connecting means during the motion of the vessel, after the stent (1) is deployed.

2. A stent (1) as claimed in claim 1, **characterized in that** said separation occurs after a period of time after implantation of the stent (1) in the vessel, the period of time being sufficient to permit neointima formation around the stent (1) in an amount sufficient to secure said stent segments (2) with respect to the vessel.

3. A stent (1) as claimed in claim 1 or 2, **characterized in that** the detachable connecting means comprises at least one designated detachment strut (4) having a cross-sectional area sufficiently low so that the designated detachment strut (4) will separate preferentially under stress placed on the stent (1) after implantation.

4. A stent (1) as claimed in claim 1 or 2, **characterized in that** the detachable connecting means comprises at least one designated detachment strut (4) made of a material that is sufficiently weaker than elsewhere in the stent (1) so that the designated detachment strut (4) will separate preferentially under stress placed on the stent (1) after implantation.

5. A stent (1) as claimed in claim 1or 2, **characterized in that** the detachable connecting means comprises at least one designated detachment strut (4) in a designated detachment zone of the stent (1), the number of designated detachment struts (4) in said designated detachment zone being less than the number of struts (4) that traverse a plane that crosses one of said stent segments (2) perpendicular to an axis of the stent segment.

## Patentansprüche

1. Stent (1) zur Implantation in ein Gefäß, wobei der Stent (1) eine Mehrzahl von Stentsegmenten (2) und Mittel zur Verbindung benachbarter Stentsegmente aufweist, **dadurch gekennzeichnet, dass** die Verbindungsmittel trennbar sind, wobei die trennbaren Verbindungsmittel zumindest eine vorgesehene Trennstrebe (4) aufweisen, wobei die vorgesehene Trennstrebe (4) eine Querschnittsfläche aufweist, die kleiner ist als die Querschnittsfläche einer Komponente (5) innerhalb des Stentsegments (2) und/oder die vorgesehene Trennstrebe (4) aus einem Material hergestellt ist, welches schwächer ist als das Material einer Komponente (5) innerhalb des Stentsegments (2), so dass sich die benachbarten Stentsegmente (2) voneinander trennen, aufgrund der physiologischen Belastung, die auf die trennbaren Verbindungsmittel während der Bewegung des Gefäßes ausgeübt wird, nachdem der Stent (1) eingesetzt ist.

2. Stent (1) gemäß Anspruch 1, **dadurch gekennzeichnet, dass** die Trennung nach einer Zeitspanne nach der Implantation des Stents (1) in das Gefäß auftritt, wobei die Zeitspanne ausreichend ist, um die Neointimaausbildung um den Stent (1) zu gestatten, in einem Maß, das ausreichend ist, um die Stentsegmente (2) bezüglich des Gefäßes zu sichern.

3. Stent (1) gemäß Anspruch 1 oder 2, **dadurch gekennzeichnet, dass** die trennbaren Verbindungsmittel zumindest eine vorgesehene Trennstrebe (4) aufweisen, die eine Querschnittsfläche aufweist, welche ausreichend gering ist, so dass die vorgesehene Trennstrebe (4) sich vorzugsweise unter der Belastung teilt, welche nach der Implantation auf den Stent (1) ausgeübt wird.

4. Stent (1) gemäß Anspruch 1 oder 2, **dadurch gekennzeichnet, dass** die trennbaren Verbindungsmittel zumindest eine vorgesehene Trennstrebe (4) aufweisen, welche aus einem Material hergestellt ist, welches ausreichend schwächer ist, als sonst wo in dem Stent (1), so dass die vorgesehene Trennstrebe (4) sich vorzugsweise unter der Belastung teilt, die nach der Implantation auf den Stent (1) ausgeübt wird.

5. Stent (1) gemäß Anspruch 1 oder 2, **dadurch gekennzeichnet, dass** die trennbaren Verbindungsmittel zumindest eine vorgesehene Trennstrebe (4) aufweisen, in einer vorgesehenen Trennzone des Stents (1), wobei die Anzahl der vorgesehenen Trennstreben (4) in der vorgesehenen Trennzone kleiner ist als die Anzahl der Streben (4), die eine Ebene durchqueren, welche eines der Stentsegmente senkrecht zu einer Achse des Stentsegments kreuzt.

## Revendications

1. Stent (1) destiné à être implanté dans un vaisseau, le stent (1) comprenant une pluralité de segments (2) et des moyens pour le raccordement des segments de stent adjacents,
**caractérisé en ce que**
lesdits moyens de raccordement sont détachables, et dans lequel lesdits moyens de raccordement détachables comprennent une entretoise de détachement désignée (4), dans lequel l'entretoise de détachement désignée (4) possède une surface en coupe transversale qui est inférieure à la surface en coupe transversale d'un composant (5) dans l'un desdits segments (2) du stent et/ou l'entretoise de détachement désignée (4) est réalisée en un matériau qui est plus faible que le matériau d'un composant (5) situé dans l'un desdits segments (2) du stent, de sorte que lesdits segments adjacents (2) du stent se séparent l'un de l'autre en réponse à une contrainte physiologique appliquée auxdits moyens de raccordement détachables pendant le déplacement du vaisseau, après que le stent (1) a été déployé.

2. Stent (1) selon la revendication 1, **caractérisé en ce que** ladite séparation apparaît au bout d'un intervalle de temps après l'implantation du stent (1) dans le vaisseau, l'intervalle de temps étant suffisant pour permettre la formation d'une nouvelle tunique interne autour du stent (1) en une quantité suffisante pour fixer lesdits segments (2) du stent par rapport au vaisseau.

3. Stent (1) selon la revendication 1 ou 2, **caractérisé en ce que** les moyens de raccordement détachables comprennent au moins une entretoise de détachement désignée (4) possédant une section transversale suffisamment faible pour que l'entretoise de détachement désignée (4) se sépare de façon préférentielle sous une contrainte appliquée au stent (1) après l'implantation.

4. Stent (1) selon la revendication 1 ou 2, **caractérisé en ce que** les moyens de raccordement amovibles comprennent au moins une entretoise de détachement désignée (4) formée d'un matériau qui est suffisamment plus faible qu'ailleurs dans le stent (1) de telle sorte que l'entretoise de détachement désignée (4) se sépare de façon préférentielle sous l'action d'une contrainte appliquée au stent (1) après l'implantation.

5. Stent (1) selon la revendication 1 ou 2, **caractérisé en ce que** les moyens de raccordement détachables comprennent une entretoise de détachement désignée (4) dans une zone de détachement désignée du stent (1), le nombre d'entretoises de détachement désignées (4) dans ladite zone de détachement désignée étant inférieure au nombre d'entretoises (4) qui croisent l'un desdits segments (2) du stent perpendiculairement à un axe du segment du stent.
